(19) Europäisches Patentamt
European Patent Office
Office européen des brevets

(11) Veröffentlichungsnummer: 0 371 255 A1

(12) # EUROPÄISCHE PATENTANMELDUNG

(21) Anmeldenummer: 89119804.6

(51) Int. Cl.5: C07C 45/71, C07C 49/84

(22) Anmeldetag: 25.10.89

(30) Priorität: 02.11.88 DE 3837116

(43) Veröffentlichungstag der Anmeldung: 06.06.90 Patentblatt 90/23

(84) Benannte Vertragsstaaten: CH DE FR GB IT LI

(71) Anmelder: BASF Aktiengesellschaft
Carl-Bosch-Strasse 38
D-6700 Ludwigshafen(DE)

(72) Erfinder: Aumueller, Alexander, Dr.
An der Marlach 5
D-6705 Deidesheim(DE)
Erfinder: Neumann, Peter, Dr.
Poststrasse 28
D-6800 Mannheim 31(DE)

(54) **Verfahren zur Herstellung von 2-Hydroxy-4-alkoxybenzophenonen.**

(57) Verfahren zur Herstellung von Alkoxybenzophenonen der Formel (I)

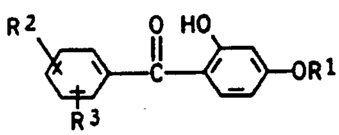

(I),

in der $R^1$ $C_1$-$C_4$-Alkyl und $R^2$ und $R^3$ unabhängig voneinander jeweils Wasserstoff, Hydroxy, $C_1$-$C_8$-Alkoxy, $C_1$-$C_4$-Alkyl oder Halogen bedeuten, durch Umsetzen von Benzophenonen der Formel (II)

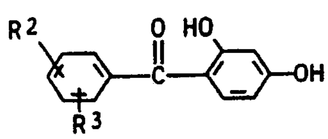

(II)

mit Dialkylsulfaten der Formel (III)

$(R^1O)_2SO_2$ (III),

wobei in den Formeln $R^1$, $R^2$ und $R^3$ die obengenannten Bedeutungen besitzen, bei dem man die Umsetzung in Wasser als Reaktionsmedium in Gegenwart einer Base vornimmt und das Dialkylsulfat (III) portionsweise zusetzt.

Nach dem Verfahren erhält man (I) in hoher Ausbeute und praktisch frei von 2,4-Dialkoxybenzophenonen.

EP 0 371 255 A1

## Verfahren zur Herstellung von 2-Hydroxy-4-alkoxybenzophenonen

2-Hydroxy-4-alkoxybenzophenone werden als Lichtschutzmittel für Kunststoffe wie PVC, Polyester, Polyacrylate, Polyolefine z.B. Polypropylen, Polyethylen, für Lacke auf der Basis von Acrylaten, Epoxidharzen oder Polyurethanen angewendet.

Weiterhin werden diese Benzophenone in Kosmetikartikeln als Schutzmittel für die Produkte und in Sonnenschutzmitteln als UV-Absorber verwendet.

Aufgabe der Erfindung war es, ein umweltfreundliches und sicher durchführbares Verfahren zur Herstellung von 2-Hydroxy-4-alkoxybenzophenonen auf der Basis der gut zugänglichen 2,4-Dihydroxybenzophenone bereitzustellen. Diese Aufgabe wird mit Hilfe des Verfahrens der Erfindung gelöst.

Dementsprechend betrifft die Erfindung ein Verfahren zur Herstellung von Alkoxybenzophenonen der Formel (I)

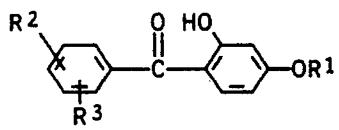
(I),

in der
$R^1$ $C_1$-$C_4$-Alkyl und
$R^2$ und $R^3$ unabhängig voneinander jeweils Wasserstoff, Hydroxy, $C_1$-$C_8$-Alkoxy, $C_1$-$C_4$-Alkyl oder Halogen bedeuten, durch Umsetzen von Benzophenonen der Formel (II)

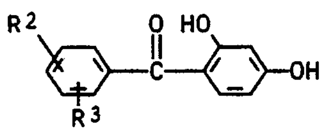
(II)

mit Dialkylsulfaten der Formel (III)
$(R^1O)_2SO_2$ (III),
wobei in den Formeln $R^1$, $R^2$ und $R^3$ die obengenannten Bedeutungen besitzen, das dadurch gekennzeichnet ist, daß man die Umsetzung in Wasser als Reaktionsmedium in Gegenwart einer Base vornimmt und das Dialkylsulfat (III) portionsweise zusetzt.

Bei dem Verfahren werden praktisch nur d.h. zu 95 % und mehr die 4-ständigen OH-Gruppen alkyliert. Die Verfahrensprodukte werden in hoher Reinheit erhalten.

Weiterhin hat das Verfahren den Vorteil, daß auch wasserfeuchte 2,4-Dihydroxybenzophenone (II), wie sie bei einigen Herstellverfahren anfallen, direkt ohne Trocknung verwendet werden können.

Als $C_1$-$C_4$-Alkyl für $R^1$, $R^2$ und $R^3$ sind im einzelnen z.B. Methyl, Ethyl, Propyl und Butyl zu nennen. Vorzugsweise steht $R^1$ für Ethyl, insbesondere für Methyl.

$R^2$ und $R^3$ können außerdem z.B. Halogen wie Chlor, Fluor, oder Brom; $C_1$-$C_8$-Alkoxy wie Methoxy, Ethoxy, Propoxy, Butoxy, Pentoxy, Hexoxy, Heptoxy, Octoxy, i-Octoxy, 2-Ethylhexoxy; Hydroxy oder Wasserstoff sein, wobei $R^2$ und $R^3$ gleich oder verschieden sein können. Für $R^2$ und $R^3$ sind als Substituenten Methoxy, Hydroxy und Wasserstoff bevorzugt, von denen Wasserstoff besonders bevorzugt ist.

Von den Benzophenonen (II) sind solche der Formeln (IIa) bis (IIc):

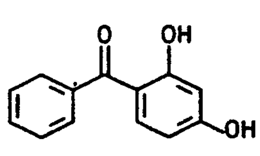
(IIa)
,
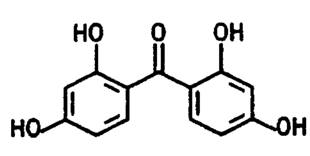
(IIb)
und
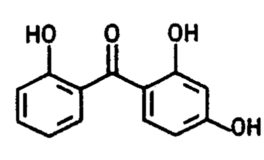
(IIc)

bevorzugt.

Trägt das Benzophenon (II) als $R^2$ oder $R^3$ eine zweite para-ständige Hydroxygruppe, wird diese beim

Verfahren der Erfindung ebenfalls alkyliert. D.h. die Mengen an (III) und Base müssen verdoppelt werden.

Das Verfahren gemäß der Erfindung wird in der Regel so durchgeführt, daß das Benzophenon (II) in Wasser in Gegenwart der Base suspendiert bis gelöst wird und dann zu diesem Gemisch bei Raumtemperatur das Dialkylsulfat (III) portionsweise zugegeben wird. Nach Beendigung der Reaktion wird das ausgefallene Alkylierungsprodukt z.B. durch Absaugen isoliert und mit Wasser gewaschen.

Gibt man die gesamte Menge des Dialkylsulfats auf einmal zu, so erhält man ein Produkt mit deutlich geringerer Reinheit und in geringerer Ausbeute.

Bei Benzophenonen (II), die eine paraständige Hydroxygruppe tragen, werden je Mol (11) 0,9 bis 1,5 Mol, vorzugsweise 1 bis 1,2 Mol des Dialkylsulfats (III) angewendet. Besonders bevorzugt sind Mengen von 1,1 Mol (III) je Mol (II) (d.h. je Äquival paraständiger Hydroxygruppe im Benzophenon).

Die Mengen an der Base sind äquivalent zu den angewendeten Mengen an (III), d.h. je Mol (III) werden 1 Äquivalent Base angewendet. Im Falle von Natronlauge oder Kalilauge werden je Mol (III) 1 Mol dieser Basen angewendet. Für den Fall, daß das Benzophenon (II) zwei paraständige Hydroxygruppen trägt, müssen je Mol (II) jeweils die doppelte Mengen an (III) und der Base angewendet werden.

Als Basen kommen die Alkalimetallhydroxide, die Alkalimetallcarbonate und Alkalihydrogencarbonate, Magnesiumhydroxid und Calciumcarbonat in Betracht, z.B. Natriumhydroxid, Kaliumhydroxid, Lithiumhydroxid, Natriumcarbonat, Natriumhydrogencarbonat, Kaliumcarbonat und -hydrogencarbonat.

Als Basen sind Natriumhydroxid und Kaliumhydroxid in Form ihrer wäßrigen Lösungen bevorzugt.

Das Dialkylsulfat (III) wird dem Reaktionsgemisch in Portionen zugegeben, vorzugsweise in 3 bis 5, insbesondere in 4, Portionen.

Dabei können die Portionen gleich oder verschieden groß sein. Vorzugsweise sind die ersten 2 bis 4 Portionen ungefähr gleich groß und die letzte bis die letzten 2 Portionen sind ungefähr die Hälfte bis ein Viertel der ersten zwei bis vier Portionen. Die Teilmengen (Portionen) werden in Abständen von 5 Minuten bis 3 h, vorzugsweise in Abständen von 15 bis 60 Minuten zugegeben. Die Reaktion erfolgt bei Temperaturen im Bereich von 0 bis 60, vorzugsweise von 15 bis 50 °C. Besonders bevorzugt ist der Bereich von 20 bis 40 °C. Die Reaktion ist im allgemeinen je nach der Größe des Ansatzes in 1 bis 24 h, bevorzugt in 2 bis 7 h beendet.

Am Ende der Reaktion evtl. noch vorhandenes Dialkylsulfat kann durch Zugeben von Ammoniak oder Ammoniakwasser zerstört werden. Zur vollständigen Fällung des Verfahrensproduktes kann es vorteilhaft sein, das Reaktionsgemisch leicht sauer zu stellen.

Die Isolierung der Verfahrensprodukte erfolgt in üblicher Weise, z.B. durch Absaugen und Waschen mit Wasser.

Das erhaltene rohe Verfahrensprodukt enthält in der Regel $< 5$ Gew.%, in den meisten Fällen $< 2,5$ Gew.% an der 2,4-Dialkoxyverbindung.

Das rohe Verfahrensprodukt kann nach bekannten Verfahren, z.B. durch Umkristallisation noch weiter gereinigt werden. Das Verfahren soll durch folgendes Ausführungsbeispiel zusätzlich erläutert werden.

Beispiel

Zu einer Mischung aus 500 ml 2n-Natronlauge und 150 ml Wasser wurden bei Raumtemperatur 214 g (= 1.0 Mol) 2,4-Dihydroxybenzophenoen gegeben und gerührt bis alles gelöst war (20 min.). Anschließend wurden 31,6 ml (= 0.33 Mol) Dimethylsulfat zugegeben und 0,5 h gerührt, dann wurden unter Rühren weitere 31,6 ml (= 0.33 Mol) Dimethylsulfat zugegeben. Nach 0,5 h wurden unter Rühren weitere 31,6 ml (= 0.33 Mol) Dimethylsulfat zugefügt und nach 0,5 h 50 ml 2n-Natronlauge und 9,5 ml Dimethylsulfat zugesetzt. Das Gemisch wurde 1 h bei Raumtemperatur gerührt und nach der Zugabe von 30 ml konzentrierter Ammoniaklösung noch 0,5 h gerührt. Der augefallene Niederschlag wurde abgesaugt und mit wenig Wasser gewaschen. Ausbeute: 349 g ($\widehat{=}$, 202,4 g trocken = 89 % d. Th.) feuchtes 2-Hydroxy-4-methoxy-benzophenon, das 1,07 % 2,4-Dimethoxybenzophenon enthält. Das feuchte Nutschgut wurde aus 1170 ml Methanol unter Zugabe von 5 g Aktivkohle umkristallisiert. Nach dem Absaugen und Trocknen im Wasserstrahlvakuum bei 50 °C wurden 171 g 75 d. Th.) reines 2-Hydroxy-4-methoxy-benzophenon erhalten. Schmp. 62 bis 63 °C. Nach dem GC enthält das Produkt kein Dimethoxyderivat.

## Ansprüche

1. Verfahren zur Herstellung von Alkoxybenzophenonen der Formel (I)

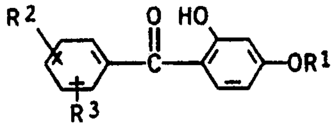

(I),

in der

$R^1$ $C_1$-$C_4$-Alkyl und

$R^2$ und $R^3$ unabhängig voneinander jeweils Wasserstoff, Hydroxy, $C_1$-$C_8$-Alkoxy, $C_1$-$C_4$-Alkyl oder Halogen bedeuten, durch Umsetzen von Benzophenonen der Formel (II)

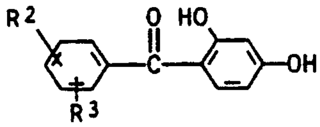

(II)

mit Dialkylsulfaten der Formel (III)

$(R^1O)_2SO_2$ (III),

wobei in den Formeln $R^1$, $R^2$ und $R^3$ die obengenannten Bedeutungen besitzen, dadurch gekennzeichnet, daß man die Umsetzung in Wasser als Reaktionsmedium in Gegenwart einer Base vornimmt und das Dialkylsulfat (III) portionsweise zusetzt.

2. Verfahren gemäß Anspruch 1, dadurch gekennzeichnet, daß man als Base ein Alkalihydroxid verwendet. 3. Verfahren gemäß Anspruch 1 oder 2, dadurch gekennzeichnet, daß man die Umsetzung bei 15 bis 50 °C vornimmt.

Europäisches Patentamt

# EUROPÄISCHER RECHERCHENBERICHT

Nummer der Anmeldung

EP 89 11 9804

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (Int. Cl.5) |
|---|---|---|---|
| X,Y | GB-A- 823 544 (WARD BLENKINSOP & CO. LTD)<br>* Beispiel III; Patentansprüche 9-11 * | 1-3 | C 07 C 45/71<br>C 07 C 49/84 |
| Y | US-A-2 357 985 (E.S. WALLIS et al.)<br>* Seite 2, Zeilen 29-52 * | 1-3 | |
| Y | DE-A-2 365 379 (MERCK & CO. INC.)<br>* Seite 71, Beispiel 38 * | 1-3 | |
| Y | US-A-3 126 414 (S.M. SPATZ et al.)<br>* Spalte 5, Zeilen 4-23 * | 1-3 | |
| A | CHEMICAL ABSTRACTS, Band 68, 1968, Seite 1206, Spalte 2, Zusammenfassung Nr. 12711z, Columbus, Ohio, US; & PL-A-52 944 (INSTYTUT PRZEMYSLU ORGANICZNEGO) 18-03-1967 | 1 | |

| RECHERCHIERTE SACHGEBIETE (Int. Cl.5) |
|---|
| C 07 C 45/00<br>C 07 C 49/00 |

Der vorliegende Recherchenbericht wurde für alle Patentansprüche erstellt

| Recherchenort | Abschlußdatum der Recherche | Prüfer |
|---|---|---|
| DEN HAAG | 01-03-1990 | BONNEVALLE E.I.H. |

**KATEGORIE DER GENANNTEN DOKUMENTE**

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus andern Gründen angeführtes Dokument

& : Mitglied der gleichen Patentfamilie, übereinstimmendes Dokument

EPO FORM 1503 03.82 (P0403)